# EUROPEAN PATENT APPLICATION

(11) **EP 0 623 310 A2**
(43) Date of publication of application: **09.11.1994**
(21) Application number: 94106908.0
(22) Date of filing: 03.05.1994
(51) Int. Cl.: A61B 17/06

(54) **Foldable package for surgical products**

(30) Priority: 05.05.1993 BR 9301757
(71) Applicant: ETHICON INC., Somerville New Jersey 08876 (US)
(72) Inventor: Bordignon, Marcos André, Sao José dos Campos, Sao Paulo (BR); Januzelli, José Lucio Leite, Sao José dos Campos, Sao Paulo (BR)
(74) Representative: Strehl Schübel-Hopf Groening & Partner

(57) **Abstract**

The present invention refers to a foldable package (100, 200) for surgical products, composed of a plurality of foldable panels (101-106) and particularly suitable for holding a surgical suture (115) wound in a non-crossed over manner on pins (111) that penetrate orifices (110; 201, 202) in a first retaining panel (101) and preferably arranged along semi-circular paths. In this manner, even a suture (115) having a large memory may be held for long periods of time in the package of this invention without taking up an accentuatedly wavy shape after withdrawal from the package (100, 200).

Further according to the present invention, the package includes a tongue-slot type locking means (130, 131) for maintaining the package (100, 200) folded and closed, the respective slot (131)being arranged transversally half way along the first retaining panel (101).

## Description

The present invention refers to a foldable package or retainer for surgical products, and more specifically to a package composed of a plurality of foldable panels particularly suitable for retaining a sterilized surgical suture made of polymeric material having elastic memory, one or more needles being fixed at its ends.

Various types of surgical suture packages are already known in the art, which vary in accordance with the nature of the suture and its end use. In general, the principal function of such packages is to protect the suture and the respective needle(s) during transport, handling and storage prior to their reaching the end user. Apart from this, the most improved packages known in the art have reduced production costs and their structural concept facilitates opening which permits the suture-needle combination to be easily reached and removed from the package.

The prior art considered most pertinent to the invention is that disclosed in Brazilian Patent PI 8201615 in the name of Johnson & Johnson, the contents of which are incorporated herein by way of reference.

That patent refers to a foldable package or retainer for a surgical suture with a needle that includes first and second foldably connected surgical product retaining panels and first and second locking panels that are also foldable connected to each other, the first locking panel being foldably connected to the first retaining panel. All such panels are rectangular, normally made of paper card and connected to each other along their longitudinal longer edges, the final configuration of the retainer or package being obtained when the retaining panels are folded over each other with the suture therebetween, and the locking panels are successively folded so as completely surround the retaining panels.

Preferably, the prior art package comprises two tongue-slot type locking devices: a first one being capable of maintaining the retaining panels folded over each other with a view to immobilizing the suture, the tongue thereof being arranged on the longitudinal free edge of the second retaining panel and the slot of which is positioned on the first retaining panel, parallel and adjacent to the edge joining the latter panel to the first locking panel.

The second locking means permits fixing of the second locking panel over the rear face of the first retaining panel, so as to maintain the package closed, and comprises a tongue arranged at the longitudinal free edge of the second locking panel, which cooperates with a slot also formed in the first retaining panel, such slot being adjacent and parallel to the slot of the first locking means.

The package disclosed in Brazilian Patent PI 8201615 is so shaped that the respective suture may be reached and withdrawn from the package by the user by partially tearing the first locking panel. Apart from this, the corresponding needle is maintained in a separate compartment from the suture which not only avoids the accidental cutting of the latter, but also possible damage to the sharp edges of the needle.

However, bearing in mind the nature and position of the above described second locking means, the prior art teaches that the suture should be wound in an over crossed manner, as in an "8", so that it is not unduly retained by the second locking means when it is removed from the package. Since all sutures have a certain degree of elastic memory, sutures that are wound in an crossed over manner for some time in the prior art package have a strong tendency, after removal from the package, not to be straight, as would be desirable, but rather accentuatedly wavy, which severely prejudices the use in practice of the suture.

In spite of this problem occurring with sutures made of the most variable materials and even those of animal (cat gut, for example), the above mentioned tendency of assuming a wavy configuration will be more pronounced in sutures made of multi- or mono-filament synthetic material which has a greater degree of elastic memory . Monofilament sutures made of polymers have a particularly large elastic memory.

One of the objects of the present invention is, therefore, to provide a foldable package containing a filamentary surgical product, such as, for example, a suture, that is wound in such a way that its configuration, after removal from the package, is as straight as possible.

Another object of this invention is to provide a surgical suture package made of foldable panels fixed to each other and shaped so that the removal of the suture may be effected without any possibility of undue retention by a locking means of the panels, independently of the manner in which the suture is would.

The above objects are attained, according to a first aspect of this invention, by means of a foldable package for surgical products, particularly adapted for retaining a filamentary surgical product, comprises first and second elongate retaining panels foldably joined to each other along their longitudinal edges, the filamentary surgical product being in a non-crossed over manner on the first retaining panel; a first auxiliary retaining panel foldably joined to a transverse edge of the first retaining panel; and a first elongate locking panel a longitudinal edge of which is foldably joined to the longitudinal edge of the first retaining panel that is opposite to the second retaining panel, the first locking panel having a substantially transverse line of weakening.

A second aspect of the present invention relates to a foldable package for surgical products, particularly adapted to retain a filamentary surgical product, comprises first and second retaining panels foldably joined to each other along their edges, the filamentary surgical product being would in a non-crossed over manner on the first retaining panel; a first locking panel an edge of which is foldably joined to the edge of the first retaining panel opposite the second retaining panel; and a tongue-slot type locking means, the respective slot being arranged transversally half-way along the first retaining panel.

According to a third aspect of this invention, a foldable package for surgical products, particularly adapted for retaining a filamentary surgical suture, comprises first and second retaining panels foldably joined along their edges, the first retaining panel having orifices arranged along substantially semi-circular paths, through which pins may pass for facilitating the winding of the filamentary surgical product in a non-crossed over manner. The present invention will now be described in greater detail with reference to the accompanying drawings, in which:
Figure 1 schematically illustrates a foldable surgical suture package as taught by the most pertinent art prior to the present invention, in an open configuration, that is to say, unfolded and laid out flat;
Figures 2 to 6 schematically show the successive stages of folding the panels that comprise the package illustrated in Figure 1;
Figure 7 illustrates a first embodiment of the foldable package for surgical products of the present invention, in an open configuration, that is to say, unfolded and laid out flat. This embodiment is particularly adequate for packing surgical sutures having a large degree of elastic memory; and
Figure 8 illustrates a second embodiment of the foldable package for surgical sutures of this invention, also when open. This embodiment is suitable for packing surgical sutures having any degree of elastic memory, preferably mullet-filament sutures.

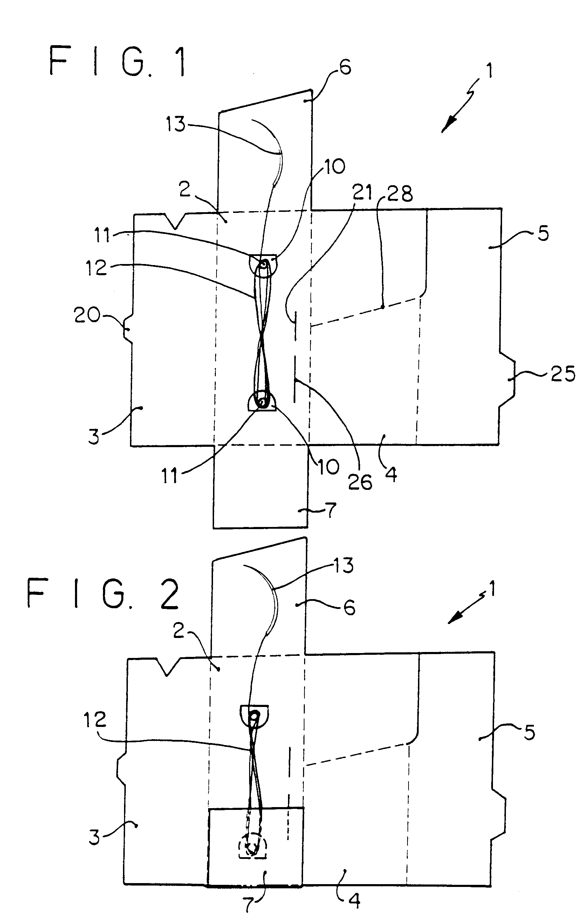

With reference to Figure 1, the foldable prior art package 1 basically comprises a first rectangular retaining panel 2 respectively foldable joined along its longer longitudinal edges to a second retaining panel 3 and a first locking panel 4, both of the latter also being rectangular. This first locking panel 4 is also foldable joined along its longitudinal edge to a second rectangular locking panel 5.

The package 1 normally also includes first and second auxiliary approximately square retaining panels 6, 7 that are respectively foldable joined to the shorter transverse edges of the first retaining panel 2.

The first retaming panel 2 has two orifices 10 arranged along the longitudinal axis of symmetry of the former, through which respective pins 11 are introduced, so as to facilitate manual winding of a suture 12 having a needle 13 fixed to one of its ends.

Suture 12 is generally made of moon-filament or woven mullet-filament polymeric material, and therefore has elastic memory. Thus, when suture 12 is freed, it tends to return to its prior configuration. Therefore, according to the art to the present invention, the winding of the suture 12 has to be in a crossed over manner, in "8", for the reasons already mentioned and which will now be described in greater detail.

Alter the manual winding step, the pins 11 are removed from orifices 10 and the suture 12 is held on the first retaining panel 2 while the needle 13 is arranged on the first auxiliary retaining panel 6 at the start of the folding and closure steps of package 1, now to be described with reference to Figures 2 to 6.

Two tongue-slot type locking means are also provided, which maintain the package 1 closed after folding of panels 2 to 7.

The first of such means is comprised of a first tongue 20 provided at the longitudinal free edge of the second retaining panel 3, and of a first slot 21, on the first retaining panel 2, in a position parallel and adjacent to the longitudinal joining edge of such panel 2 with the first locking panel 4.

The second locking means comprises a second tongue 24, situated at the free edge of the second locking panel 5, which cooperates with a second slot 26, also provided in the first retaining panel 2, in a position parallel and adjacent to the first slot 21.

Panels 2 to 7 which form the package 1 are normally made of paper card of any weight. However, they may be made of films of plastic or other flexible material.

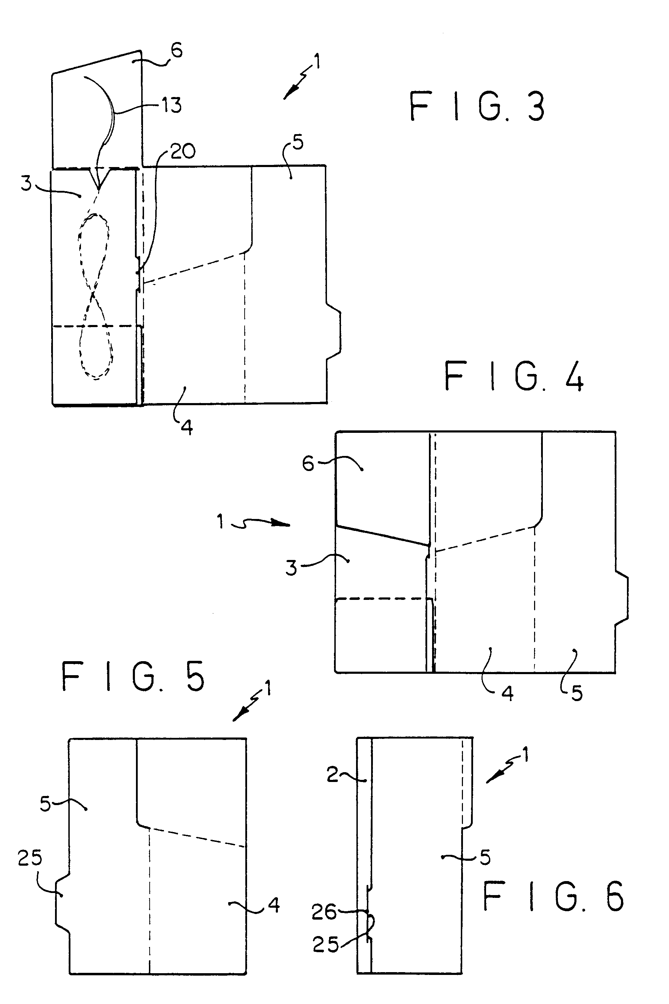

Figures 2 to 6 show the successive stages of folding and closing the package 1. Firstly, as shown in Figure 2, the second auxiliary retaining panel 7 is folded over suture 12 and first retaining panel 2, the second retaining panel 3 then being folded over suture 12, first retaining panel 2 and second auxiliary retaining panel 7, as shown in Figure 3. Such panels 2, 3 and 7 are held in that position after the insertion of first tongue 20 in first slot 21.

First locking panel 4 is then folded over second retaining panel 3, with needle 13 and part of suture 12 between them (Figure 4), and the first and second locking panels 4, 5 are successively folded over the other panels, so that the second tongue 25 is finally inserted into the second slot 26 behind the first retaining panel 2, thus completing mounting of the package 1 (Figures 5 and 6).

Needle 13 contained in package 1 when closed as described above is maintained in a separate compartment from suture 12 and is easily accessible due to the fact that part of the first locking panel 4 may be lifted after tearing a weakening line 28, permitting the first auxiliary retaining panel 6 also to be lifted, so as to expose needle 13 and allow a user to catch it with his hand or with tweezers for pulling the entire suture 12 from package 1.

As already mentioned, all sutures and especially mono-filament sutures made of polymeric material have elastic memory. For this reason the turns of a suture wound around pins 11 tend to expand centrifugally, that is to say, in the direction of the edges of the first retaining panel 2, creating a considerable risk of the second tongue 25 snagging on one or more of such turns when it is inserted into the second slot 26. In such case, suture 12 would be undesirably caught on being pulled out of package 1. This snagging can have disastrous consequences during a delicate surgical procedure.

In view of this tendency of centrifugal expansion and the positioning of the second tongue 25 and of the second slot 26 in the package 1 made in accordance with the teachings of the prior art, suture 12 must necessarily be wound in a crossed over manner, forming an "8", so as to reduce the centrifugal expansion of the turns of the suture 12 to a minimum, thus avoiding it being immobilized by the second tongue 25 when the latter is inserted in the second slot 26.

However, the sutures 12 packed in packages 1 of the above described type remain wound in the form of an "8" during relatively long periods until they are finally withdrawn from the respective packages 1 for use.

For this reason, the above mentioned elastic memory of suture 12 will cause it to have a strong tendency to assume an accetuated waviness after removal from package 1 and this makes handling by the user difficult.

Consequently, it being desirable in the art for sutures to be straight when withdrawn from the corresponding package and left in repose, applicant carried out research to discover that sutures wound in a non-crossed over manner, that is to say, in a circular or oval configuration, in the respective packages tend to be substantially less wavy after withdrawal from such packages.

On the other hand, due to the above mentioned tendency to centrifugal expansion of sutures wound in a circular or oval manner, when held in packages made in accordance with the teachings of the prior art, there will be the danger of one or more portions of the sutures to be undesirably caught by the second tongue 25 when it is inserted into the second slot 26, this making withdrawal of suture 12 from package 1 difficult. Also as mentioned above, a greater degree of centrifugal expansion was observed with sutures of synthetic material, especially with those that are mono-filamentary and made of polymers.

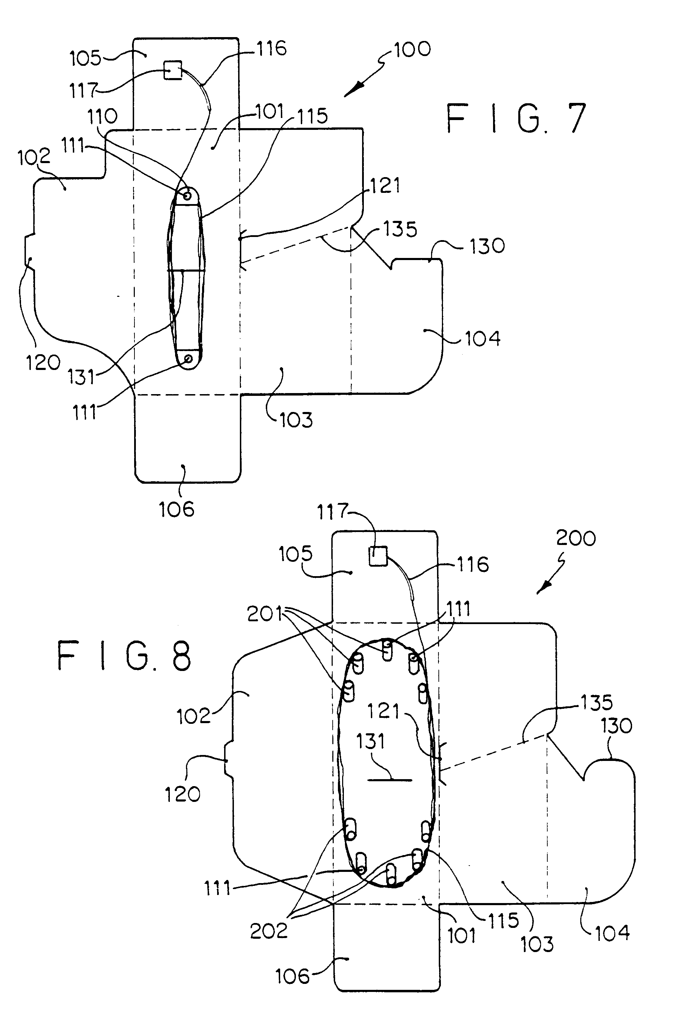

According to the present invention, this problem is solved by means of an improved foldable package, an embodiment of which is shown by way of example in the open position, that is to say, unfolded and laid out flat, in Figure 7' it being indicated generally by reference number 100.

Due to the novel structural concept of package 100, a suture wound ovally may be maintained therein without risk of undesirable snagging on its removal.

Furthermore, as already mentioned, this embodiment of the foldable package 100 is particularly suitable for packing sutures having large elastic memory, as for example, those made of monofilament polymeric material, for example, nylon, polyester, polyethylene and polypropylene. Package 100 comprises a first rectangular retaining panel 101 respectively and foldable joined along its longer longitudinal edges to a second retaining panel 102 and to a first locking panel 103, both of which are also rectangular. This latter panel 103 is also foldably joined along its longitudinal edge to a second locking panel 104.

According to a preferred embodiment of the invention, such second locking panel 104 has a reduced longitudinal dimension when compared with the corresponding dimensions of the other panels 101 to 103.

Package 100 further includes first and second approximately square auxiliary retaining panels 105 and 106 that are foldably connected along their shorter transverse edges to the first retaining panel 101.

Panels 101 to 106 that form package 100 are preferably made of paper card of any weight so that the final cost thereof may be maintained at a minimum. Naturally, such panels may also be made of films of plastic, metal (aluminium) or any other flexible material. Apart from this, it is emphasized that the presence of the second auxiliary retaining panel 106 is optional and that it could be eliminated so as further to reduce the final cost of package 100.

In the presently described embodiment of package 100, two orifices 110 are provided in the first retaining panel 101, through which two pins 111 are introduced, the purpose of which is to facilitate the manual winding of a suture 115 having a needle 116 fixed to one of its ends. Optionally suture 115 may have a needle attached to each of its ends, or it may even not have any needle at all. Apart from this, package 100 may contain more than one suture.

According to this invention and as illustrated in Figure 7, suture 115 is wound in a non-crossed over fashion around pins 111 so that the turns of the latter may assume a substantially oval shape as they expand centrifugally when freed. As already explained, this centrifugal expansion results from the elastic memory of suture 115 and it is especially large when the suture is made of monofilament polymeric material.

As also mentioned above, sutures 115 having elastic memory that are wound and packed in an oval or circular configuration (non-crossed over) tend to assume a much less wavy shape when removed from their respective packages 100, as opposed to sutures wound in a crossed over manner. This tendency makes the sutures packed in packages 100 made in accordance with the teachings of this invention much easier to use since they are substantially straight after withdrawal from the respective packages 100.

The procedure of folding the panels 101 to 106 to close and form the final package 100 is analogous to that described with relation to prior art package 1 illustrated in Figures 1 to 6. Thus, when package 100 is closed, needle 116 is advantageously maintained in a separate compartment from suture 115, thus avoiding accidental cutting or damage to the edges of the needle 116.

Generally, it is advantageous for the front face of the first auxiliary retaining panel 105 to have fixed thereto a retaining body 117 into which the needle 116 may be introduced for better immobilization thereof. According to a preferred embodiment, this body 117 is three dimensional and made of polymeric foam, although it could be made of paper card or any other resilient material.

Package 100 further includes a first locking means composed of a first tongue 120, provided at the free longitudinal edge of the second retaining panel 102, and a first slot 121 suitable for receiving first tongue 120 and formed at the edge joining the first retaining panel 101 to the first locking panel 103. The function of this first locking means 120, 121 is to maintain the second retaining panel 102 folded over the first retaining panel 101, with the suture 115 and the second auxiliary retaining panel 106 between the two, in a manner analogous to that illustrated in Figure 3.

In accordance with an optional embodiment, the second auxiliary retaining panel 106 may be folded over the second retaining panel 102, in its turn folded over the first retaining panel 101 and the suture 115.

According to the invention, a second locking means is provided, that is composed of a second tongue 130, at the free edge of the second locking panel 104 and having an axis of symmetry parallel both to the joining lines between retaining and locking panels 101-104 and to the longitudinal axis of the package 100, when closed.

The second locking means further comprises a second slot 131, substantially at the center of the first retaining panel 101, between orifices 110, and transverse of the longitudinal axis of the said first panel 101.

The second locking means 130, 131 serves to immobilize the second locking panel 104 over the back face of the first retaining panel 101, in a manner analogous to that illustrated in Figure 6, this maintain package 100 in the folded configuration. Thus, the second locking means 130, 131 makes the first locking means 120, 121 merely optional.

According to the present invention and contrary to the prior art, there is no possibility of the second tongue 130 snagging unduly on a portion of suture 115 when it penetrates the second slot 131 since, the suture 115 advantageously wound in an oval manner over the first retaining panel 102, no turn or part of the suture 115 will be located at the central region of the panel 101 due to tendency of centrifugal expansion of its turns towards the edges of panel 101, this being caused by the elastic memory of the material that normally forms the suture 115.

Although the second tongue 130 is preferably arranged with its axis of symmetry parallel to the longitudinal axis of the package 100 when closed, with the second slot 131 transverse to such axis, this invention also covers a foldable package 100 having a second tongue the axis of symmetry of which is perpendicular, or at least diagonal, with respect to the said axis of the package 100, the second slot being correspondingly positioned.

As illustrated in Figure 7, the first locking panel 103 preferably has a weakening line 135 that can easily be torn so that a portion of this first locking panel 103 and subsequently the first auxiliary retaining panel 105 can be raised by a user. In this manner, access to the suture 115 and to the needle 116 is facilitated.

Additionally, the back face of the first locking panel 103, which faces outwardly after closure of package 100, may contain information regarding the suture 115 and needle 116 (diameter, length etc.).

Figure 8 shows a second embodiment of this invention, which is suitable for holding surgical sutures of any degree of elastic memory.

As shown, package 200 has various portions identical to those that comprise the package 100 illustrated in Figure 7. Consequently, such portions are designated in Figure 8 with the same reference numbers as are used in Figure 7.

As can be seen, the difference between package 200 and that indicated by number 100 in Figure 7 and described above may be summarized by the number and arrangement of the orifices 201, 202 in the first retaining panel 101.

In order for package 200 to be able to hold surgical sutures having all degrees of elastic memory without there being any risk of any part thereof being caught unduly by the second locking means 130, 131, the turns of the suture 115 must be originally arranged along a substantially circular or oval path. This is because, in the case of sutures made of materials having smaller degrees of elastic memory, as for example those made of material of animal or vegetable origin (cat gut, silk, cotton, etc.) or woven multi-filament polymeric material (nylon, polyester, polyethylene, polypropylene, etc.), the tendency to centrifugal expansion after winding may be insufficient to ensure that the turns of the suture 115 shift themselves as far as the edges of the first retaining panel 101, leaving totally free the central portion of such panel 101 where the second slot 131 is to be found.

Thus, in accordance with the embodiment shown in Figure 8, the said orifices 201, 202 are arranged along substantially semi-circular paths in respective first and second groups adjacent the shorter transverse edges of the first retaining panel 101. In this manner, during the step of winding the suture 115, a plurality of pins 111 pass through orifices 201, 202 and facilitate the accommodation of the turns of the suture 115 along an oval path that is adjacent the edges of the first retaining panel 10, independently of a centrifugal expansion of the turns of suture 115.

Preferably, there are at least 3 (three) orifices 201, 202 in each of the said first and second groups, receptively arranged along semi-circular paths, so that suture 115 may be wound without accentuated kinks at the location it passes around pins 111.

Apart from this, the diameter of the semi-circles formed by orifices 201, 202, also according to a preferred embodiment, is only slightly less than the width of the first retaining panel 101.

It is noted that the foldable packages for surgical products described above are only preferred embodiments of the present invention, the true scope of which is defined in the following claims.

## Claims

1. A foldable package (100, 200) for surgical products, particularly adapted for retaining a surgical suture (115, 116), characterised by comprising:
first and second elongate retaining panels (101, 102) foldably joined to each other along their longitudinal edges, said suture (115, 116) being in a non-crossed over manner on said first retaining panel (101);
a first auxiliary retaining panel (105) foldably joined to a transverse edge of said first retaining panel(101 and
a first elongate locking panel (103), a longitudinal edge of which is foldably joined to the longitudinal edge of said first retaining panel (101) that is opposite to said second retaining panel (102), said first locking panel (103) having a substantially transverse line of weakening (135).

2. A foldable package according to claim 1, characterised in that said first retaining panel (101) is provided with orifices (201, 202) arranged along substantially semi-circular paths, through which pass pins (111) that facilitate manual winding of said suture (115, 116).

3. A foldable package according to claim 1, characterised in that said retaining panels (101, 102) and said first locking panel (103) are rectangular and joined to each other along their longitudinal edges, said package further comprising:
a second rectangular locking panel (104) foldably joined to the longitudinal edge of said first locking panel (103); and
a tongue-slot type locking means (130, 131), the respective slot (131) being arranged transversally half-way along said first retaining panel (101) and the respective tongue (130) being arranged at the free edge of said second locking panel (104), with its axis of symmetry substantially parallel to the longitudinal edges of said retaining panels (101, 102) and of said first locking panel (103).

4. A foldable package (100, 200) for surgical products, particularly adapted to retain a surgical suture (115, 116), characterised comprising:
first and second retaining panels (101, 102) foldably joined to each other along their edges, said suture (115, 116) being would in a non-crossed over manner on the first retaining panel (101);
a first locking panel (103) an edge of which is foldably joined to the edge of said first retaining panel (101) opposite said second retaining panel; (102) and
a tongue-slot type locking means (130, 131), the respective slot (131) being arranged transversally half-way along said first retaining panel (101).,

5. A foldable package according to claim 4, characterised in that said retaining panels (101, 102) and said first locking panel (103) are rectangular and joined to each other along their longitudinal edges, and said package additionally comprises:
a second locking panel (104) foldably joined to the longitudinal edge of said first locking panel (103), said tongue (130) of said locking means being arranged at the free edge of said second locking panel (104), with its axis of symmetry substantially parallel to the longitudinal edges of said retaining panels (101, 102) and of said first locking panel (103), and said slot (131) being transverse in relation to the longitudinal axis of said first retaining panel (103).

6. A foldable package according to claim 5, characterised in that said second locking panel (104) has a reduced longitudinal dimension compared to the longitudinal dimensions of said retaining panels (101, 102) and of said first locking panel (103).

7. A foldable package (100, 200) for surgical products, particularly adapted for holding a surgical suture (115, 116), characterised by comprising:
first and second retaining panels (101, 102) foldably joined to each other at their edges, said first retaining panel (101) having orifices (201, 202) arranged along substantially semi-circular paths, through which pass pins (111) for facilitating winding of said suture (115, 116) in a non-crossed over manner.

8. A foldable package according to claim 8, characterised in that:
said retaining panels (101, 102) and said first locking panel (103) are rectangular and joined to each other along their longitudinal edges; and
said orifices (201, 202) are arranged, in groups of at least three, along two semi-circles of which the concave portions are respectively adjacent the transverse edges of said first retaining panel (101).

9. A foldable package according to claim 8, characterised by additionally comprising:
first and second locking panels (103, 104) foldably joined to each other, said first locking panel (103) being rectangular and foldably joined along its longitudinal edge to the longitudinal edge of said first retaining panel (101), opposite said second retaining panel (102); and
a tongue-slot type locking means (130, 131), the respective slot (131) being positioned transversely half-way along said first retaining panel (101) and the respective tongue (130) being arranged at the free edge of said second locking panel (104), with its axis of symmetry substantially parallel to the longitudinal edges of said retaining panels (101, 102) and of said first locking panel (103).

10. A foldable package according to claim 9, in which said slot (131) is substantially transverse to the longitudinal axis of said first retaining panel (101).
